# EUROPEAN PATENT APPLICATION

(11) **EP 4 445 894 A1**
(43) Date of publication of application: **16.10.2024**
(21) Application number: 22904303.9
(22) Date of filing: 08.12.2022
(51) Int. Cl.: A61K 8/99, A23L 33/135, A61K 35/74, A61K 35/744, A61P 17/00, A61P 17/02, A61P 19/02, A61P 19/04, A61P 19/10, A61P 27/02, A61P 43/00, A61Q 19/00, A61Q 19/08

(54) **COLLAGEN PRODUCTION PROMOTER, HYALURONIC ACID PRODUCTION PROMOTER, AND EPIDERMAL KERATINOCYTE ACTIVATOR**

(30) Priority: 09.12.2021 JP 2021200363
(71) Applicant: Kyushu University, National University Corporation, Nishi-ku Fukuoka-shi Fukuoka 819-0395 (JP); Osaka Soda Co., Ltd., Osaka-shi Osaka 550-0011 (JP)
(72) Inventor: KATAKURA, Yoshinori, Fukuoka-shi, Fukuoka 819-0395 (JP); NISHIKAWA Kouji, Osaka-shi, Osaka 550-0011 (JP); IDOGAKI, Hideaki, Osaka-shi, Osaka 550-0011 (JP); TOKIMOTO, Yuji, Osaka-shi, Osaka 550-0011 (JP)
(74) Representative: Müller-Boré & Partner Patentanwälte PartG mbB
(86) International application number: PCT/JP2022/045317
(87) International publication number: WO 2023/106376

(57) **Abstract**

The purpose of the present invention is to provide a novel material that promotes the production of collagen and/or hyaluronic acid and/or activates human epidermal keratinocytes. A lactic acid bacterium belonging to the genus Fructobacillus, a culture of the lactic acid bacterium, a culture supernatant of the lactic acid bacterium, and/or an extract of the lactic acid bacterium are useful as active ingredient of a collagen production promoter, a hyaluronic acid production promoter, and an epidermal keratinocyte activator.

## Description

### TECHNICAL FIELD

The present invention relates to a collagen production promoter, a hyaluronic acid production promoter, and an epidermal keratinocyte activator.

### BACKGROUND ART

Collagen is widely distributed in living organisms as a component of connective tissues, skin, bones and the like. The promotion of the production of collagen is considered effective for, for example, remedying roughness, sagging, wrinkles, wounds and the like of the skin, and treating arthritis, tenosynovitis and the like. For this reason, a search for an active ingredient that promotes the production of collagen has been performed.

For example, Patent Document 1 discloses a collagen production promoter containing an extract of Phyllanthus emblica, Patent Document 2 discloses a collagen production promoter containing an extract of seeds of passion fruit as an active ingredient, and Patent Document 3 discloses a collagen production promoter made from essential oil obtained by steam distillation from roman chamomile (Anthemis nobilis).

Hyaluronic acid is widely distributed in living organisms as a component of skin, joint fluid, vitreous bodies and ligaments. The promotion of the production of hyaluronic acid is considered effective for improving the skin moisturizing effect, remedying sagging of the skin, treating arthritis, treating keratoconjunctive epithelium disorder, and retaining the anterior chamber during cataract and corneal transplant surgery, and the like. For this reason, a search for an active ingredient that promotes the production of hyaluronic acid has been performed.

For example, Patent Document 4 discloses a hyaluronic acid production promoter containing an extract of a seaweed belonging to the genus Darbilia of Durvilleaceae as an active ingredient, Patent Document 5 discloses a hyaluronic acid production promoter containing an eggshell membrane as an active ingredient, and Patent Document 6 discloses a hyaluronic acid production promoter containing a leg part of poultry or a treated product thereof.

The skin is a tissue including epidermis and dermis, and performs a barrier function of protecting the body from external physical and/or chemical stress. The epidermis present on the outer side of the skin tissue includes a basal layer, a spinous layer, a granular layer and a horny layer, and is mainly composed of epidermal keratinocytes. The epidermal keratinocytes divided in the basal layer maintain the epidermis by repeating a turnover in which the cells undergo differentiation and maturity, move to the upper layer, reach the horny layer, and then fall. The turnover of the epidermis is involved in the skin barrier function, maintenance of the moisture content, and the like. Therefore, it is considered that stagnation of the turnover of proliferation, keratinization and falling of epidermal keratinocytes by aging and/or external stress causes skin roughness and/or dry skin. For this reason, an epidermal keratinocyte activator is used for preventing and/or treating skin roughness and/or dry skin.

For example, Patent Document 7 discloses proliferation of epidermal keratinocyte containing a water extract of a flower of woodbine of the genus Lonicera of Caprifoliaceae and a water extract of a stigma of saffron of the genus Crocus of Iridaceae, and Patent Document 8 discloses an epidermal keratinocyte activator containing an extract of leaves of Camellia japonica as an active ingredient.

### PRIOR ART DOCUMENTS

### PATENT DOCUMENTS

Patent Document 1: Japanese Patent Laid-open Publication No. 2008-156287
Patent Document 2: Japanese Patent Laid-open Publication No. 2009-102299
Patent Document 3: Japanese Patent Laid-open Publication No. 2010-180179
Patent Document 4: Japanese Patent Laid-open Publication No. 9-176036
Patent Document 5: Japanese Patent Laid-open Publication No. 2014-40402
Patent Document 6: Japanese Patent Laid-open Publication No. 2016-129174
Patent Document 7: Japanese Patent Laid-open Publication No. 2018-83794
Patent Document 8: Japanese Patent Laid-open Publication No. 2012-102049

### SUMMARY OF THE INVENTION

### PROBLEMS TO BE SOLVED BY THE INVENTION

An object of the present invention is to provide a novel material that promotes the production of collagen and/or hyaluronic acid and activates epidermal keratinocytes.

### MEANS FOR SOLVING THE PROBLEM

The present inventors have discovered that a predetermined lactic acid bacterium and/or a component derived from the predetermined lactic acid bacterium have an action of promoting the production of collagen and hyaluronic acid and an effect of activating epidermal keratinocytes. The present invention has been completed by further conducting studies based on the above-mentioned findings.

That is, the present invention provides an invention of the aspects described below.

Item 1. A collagen production promoter including a lactic acid bacterium belonging to the genus Fructobacillus, a culture of the lactic acid bacterium, a culture supernatant of the lactic acid bacterium, and/or an extract of the lactic acid bacterium.

Item 2. The collagen production promoter according to item 1, in which the lactic acid bacterium is Fructobacillus fructosus.

Item 3. The collagen production promoter according to item 1 or 2, in which the lactic acid bacterium is a Fructobacillus fructosus NBRC 3516 strain.

Item 4. The collagen production promoter according to any one of items 1 to 3, including nicotinamide adenine dinucleotide and optionally including a nicotinamide mononucleotide, in which a content of the nicotinamide mononucleotide is 5 parts by weight or less per 1 part by weight of the nicotinamide adenine dinucleotide.

Item 5. A hyaluronic acid production promoter including a lactic acid bacterium belonging to the genus Fructobacillus, a culture of the lactic acid bacterium, a culture supernatant of the lactic acid bacterium, and/or an extract of the lactic acid bacterium.

Item 6. The hyaluronic acid production promoter according to item 5, in which the lactic acid bacterium is Fructobacillus fructosus.

Item 7. The hyaluronic acid production promoter according to item 5 or 6, in which the lactic acid bacterium is a Fructobacillus fructosus NBRC 3516 strain.

Item 8. The hyaluronic acid production promoter according to any one of items 5 to 7, including nicotinamide adenine dinucleotide and optionally including a nicotinamide mononucleotide, in which a content of the nicotinamide mononucleotide is 5 parts by weight or less per 1 part by weight of the nicotinamide adenine dinucleotide.

Item 9. An epidermal keratinocyte activator including a lactic acid bacterium belonging to the genus Fructobacillus, a culture of the lactic acid bacterium, a culture supernatant of the lactic acid bacterium, and/or an extract of the lactic acid bacterium.

Item 10. The epidermal keratinocyte activator according to item 9, in which the lactic acid bacterium is selected from the group consisting of Fructobacillus fructosus, Fructobacillus durionis, and Fructobacillus tropaeoil.

Item 11. The epidermal keratinocyte activator according to item 9 or 10, in which the lactic acid bacterium is selected from the group consisting of a Fructobacillus fructosus NBRC 3516 strain, a Fructobacillus durionis RD 011727 strain, a Fructobacillus tropaeoil RD 012353 strain, and a Fructobacillus tropaeoil RD 012354 strain.

Item 12. The epidermal keratinocyte activator according to any one of items 9 to 11, including nicotinamide adenine dinucleotide and optionally including a nicotinamide mononucleotide, in which a content of the nicotinamide mononucleotide is 5 parts by weight or less per 1 part by weight of the nicotinamide adenine dinucleotide.

Item 13. A food or beverage product, a cosmetic product or a pharmaceutical product including the collagen production promoter according to any one of items 1 to 4.

Item 14. A food or beverage product, a cosmetic product or a pharmaceutical product including the hyaluronic acid production promoter according to any one of items 5 to 8.

Item 15. A food or beverage product, a cosmetic product or a pharmaceutical product including the epidermal keratinocyte activator according to any one of items 9 to 12.

Item 16. An agent for preventing or remedying sagging and/or wrinkles of skin,
an agent for treating roughness and/or wounds of the skin,
an agent for preventing or treating osteoporosis, arthritis and/or tenosynovitis,
a skin moisturizing agent,
an agent for treating keratoconjunctive epithelium disorder,
an agent for retaining the anterior chamber during ophthalmic surgery,
a keratin thickening suppressing agent,
an agent for remedying skin dullness,
a skin regeneration promoting agent,
a skin-lightening agent,
a skin texture improving agent, or
an agent for remedying acne and/or pimples,
the agent including a lactic acid bacterium belonging to the genus Fructobacillus, a culture of the lactic acid bacterium, a culture supernatant of the lactic acid bacterium, and/or an extract of the lactic acid bacterium.

Item 17. Use of a lactic acid bacterium belonging to the genus Fructobacillus, a culture of the lactic acid bacterium, a culture supernatant of the lactic acid bacterium, and/or an extract of the lactic acid bacterium, for producing a collagen production promoter.

Item 18. Use of a lactic acid bacterium belonging to the genus Fructobacillus, a culture of the lactic acid bacterium, a culture supernatant of the lactic acid bacterium, and/or an extract of the lactic acid bacterium, for producing a hyaluronic acid production promoter.

Item 19. Use of a lactic acid bacterium belonging to the genus Fructobacillus, a culture of the lactic acid bacterium, a culture supernatant of the lactic acid bacterium, and/or an extract of the lactic acid bacterium, for producing an epidermal keratinocyte activator.

Item 20. Use of a composition containing a lactic acid bacterium belonging to the genus Fructobacillus, a culture of the lactic acid bacterium, a culture supernatant of the lactic acid bacterium, and/or an extract of the lactic acid bacterium, for promoting collagen production, promoting hyaluronic acid production or activating epidermal keratinocytes.

Item 21. The use according to item 17, for preventing or remedying sagging and/or wrinkles of the skin,
treating roughness and/or wounds of the skin,
preventing or treating osteoporosis, arthritis and/or tenosynovitis,
moisturizing the skin,
treating keratoconjunctive epithelium disorder,
retaining the anterior chamber during ophthalmic surgery,
suppressing keratin thickening,
remedying skin dullness,
promoting skin regeneration,
lightening the skin,
improving the skin texture, or
remedying acne and/or pimples.

Item 22. A method for promoting collagen production, the method including the step of administering to a subject in need of promotion of collagen production an effective amount of a lactic acid bacterium belonging to the genus Fructobacillus, a culture of the lactic acid bacterium, a culture supernatant of the lactic acid bacterium, and/or an extract of the lactic acid bacterium.

Item 23. A method for promoting hyaluronic acid production, the method including the step of administering to a subject in need of promotion of hyaluronic acid production an effective amount of a lactic acid bacterium belonging to the genus Fructobacillus, a culture of the lactic acid bacterium, a culture supernatant of the lactic acid bacterium, and/or an extract of the lactic acid bacterium.

Item 24. A method for activating epidermal keratinocytes, the method including the step of administering to a subject in need of activation of epidermal keratinocytes an effective amount of a lactic acid bacterium belonging to the genus Fructobacillus, a culture of the lactic acid bacterium, a culture supernatant of the lactic acid bacterium, and/or an extract of the lactic acid bacterium.

### ADVANTAGES OF THE INVENTION

According to the present invention, there is provided a novel material that is useful as an active ingredient of a collagen and/or hyaluronic acid promoter and an epidermal keratinocyte activator. Specifically, according to the present invention, a lactic acid bacterium belonging to the genus Fructobacillus, a culture of the lactic acid bacterium, a culture supernatant of the lactic acid bacterium, and/or an extract of the lactic acid bacterium are provided as an active ingredient of a collagen production promoter, a hyaluronic acid production promoter and an epidermal keratinocyte activator.

### BRIEF DESCRIPTION OF THE DRAWINGS

Fig. 1 shows an effect of a test product (including a sample A) on production of collagen per cell (Test Example 1).
Fig. 2 shows an effect of a test product (including the sample A) on production of hyaluronic acid per cell (Test Example 1).
Fig. 3 shows an effect of a test product (including a lactic acid bacterium extraction liquid A1) on production of collagen per cell (Test Example 2).
Fig. 4 shows an effect of a test product (including the lactic acid bacterium extraction liquid A1) on production of hyaluronic acid per cell (Test Example 2).
Fig. 5 shows an effect of a test product (including a sample B) on production of collagen per cell (Test Example 3).
Fig. 6 shows an effect of a test product (including the sample B) on production of hyaluronic acid per cell (Test Example 3).
Fig. 7 shows an effect of a test product (including a lactic acid bacterium extraction liquid B 1) on production of collagen per cell (Test Example 4).
Fig. 8 shows an effect of a test product (including the lactic acid bacterium extraction liquid B 1) on production of hyaluronic acid per cell (Test Example 4).

### EMBODIMENTS OF THE INVENTION

A collagen production promoter, a hyaluronic acid production promoter and an epidermal keratinocyte activator of the present invention all contain a lactic acid bacterium belonging to the genus Fructobacillus, a culture of the lactic acid bacterium, a culture supernatant of the lactic acid bacterium, and/or an extract of the lactic acid bacterium as an active ingredient. Hereinafter, the collagen production promoter, the hyaluronic acid production promoter and the epidermal keratinocyte activator will be described in detail.

### [1. Active ingredient]

The active ingredient of collagen production promoter, the hyaluronic acid production promoter and the epidermal keratinocyte activator of the present invention is a lactic acid bacterium belonging to the genus Fructobacillus, a culture of the lactic acid bacterium, a culture supernatant of the lactic acid bacterium, and/or an extract of the lactic acid bacterium.

### [1-1. Lactic acid bacterium belonging to genus Fructobacillus]

The lactic acid bacterium belonging to the genus Fructobacillus (hereinafter, also referred to as a "predetermined lactic acid bacterium") is a microorganism that produces nicotinamide mononucleotide (NMN) and/or nicotinamide adenine dinucleotide (NAD). The lactic acid bacterium belonging to the genus Fructobacillus is preferably a microorganism that produces at least NAD, and more preferably a microorganisms that produces both NMN and NAD.

Specific examples of the lactic acid bacterium belonging to the genus Fructobacillus include Fructobacillus durionis, Fructobacillus tropaeoil, and Fructobacillus fructosus, with Fructobacillus tropaeoil and Fructobacillus fructosus being more preferable.

Specific examples of the lactic acid bacterium belonging to the genus Fructobacillus include a Fructobacillus durionis RD 011727 strain, a Fructobacillus tropaeoil RD 012353 strain, a Fructobacillus tropaeoil RD 012354 strain, a Fructobacillus fructosus NBRC 3516 strain, and a Fructobacillus durionis NBRC 113239 strain.

The Fructobacillus durionis RD 011727 strain has been internationally deposited with National Institute of Technology and Evaluation Patent Microorganisms Depositary (NPMD) (Room 122, 2-5-8 Kazusakamatari, Kisarazu-shi, Chiba, Japan) under accession No. NITE BP-02764 on October 28, 2020.

The Fructobacillus tropaeoil RD 012353 strain has been internationally deposited with National Institute of Technology and Evaluation Patent Microorganisms Depositary (NPMD) (Room 122, 2-5-8 Kazusakamatari, Kisarazu-shi, Chiba, Japan) under accession No. NITE BP-02765 on October 28, 2020.

The Fructobacillus tropaeoil RD 012354 strain has been internationally deposited with National Institute of Technology and Evaluation Patent Microorganisms Depositary (NPMD) (Room 122, 2-5-8 Kazusakamatari, Kisarazu-shi, Chiba, Japan) under accession No. NITE BP-02766 on October 28, 2020.

These genus Fructobacillus lactic acid bacteria may be used alone, or may be used in combination of two or more thereof.

Among these genus Fructobacillus lactic acid bacteria, Fructobacillus tropaeoil and Fructobacillus fructosus are preferable, and Fructobacillus fructosus is more preferable, from the viewpoint of further effectively improving the effect of promoting the production of collagen and/or the effect of promoting the production of hyaluronic acid.

Among these genus Fructobacillus lactic acid bacteria, Fructobacillus durionis, Fructobacillus tropaeoil and Fructobacillus fructosus are preferable, Fructobacillus tropaeoil and Fructobacillus fructosus are more preferable, and Fructobacillus fructosus is particularly preferable, from the viewpoint of further effectively improving the effect of activating epidermal keratinocytes.

More specifically, among these genus Fructobacillus lactic acid bacteria, the Fructobacillus durionis RD 011727 strain, the Fructobacillus tropaeoil RD 012353 strain, the Fructobacillus tropaeoil RD 012354 strain and the Fructobacillus fructosus NBRC 3516 strain are preferable, the Fructobacillus tropaeoil RD 012353 strain, the Fructobacillus tropaeoil RD 012354 strain and the Fructobacillus fructosus NBRC 3516 strain are more preferable, and the Fructobacillus tropaeoil RD 012353 strain and the Fructobacillus fructosus NBRC 3516 strain are still more preferable, and the Fructobacillus fructosus NBRC 3516 strain is particularly preferable, from the viewpoint of further effectively improving the effect of activating epidermal keratinocytes.

### [1-2. Form of active ingredient]

The form of the active ingredient is at least one of the predetermined lactic acid bacterium, a culture of the lactic acid bacterium, a culture supernatant of the lactic acid bacterium, and an extract of the lactic acid bacterium. An active ingredient in any one of these forms may be used alone, or active ingredients in a plurality of forms may be used in combination.

### [1-2-1. Lactic acid bacterium]

When the active ingredient is in the form of a lactic acid bacterium, the active ingredient includes bacterial cell components of the lactic acid bacterium and a product that is or has been present in the lactic acid bacterium. The lactic acid bacterium may be either a living bacterium or a dead bacterium. In the case of a dead bacterium, bacterial cells may be crushed. In the case of a crushed bacterium, a part of the bacterial cell components may be removed by means other than extraction. Further, the lactic acid bacterium may be in the form of dried bacterial cell powder.

The lactic acid bacterium can be obtained by a production method including the steps of: culturing one or more of the predetermined lactic acid bacteria, and separating and collecting a bacterial cell. The method that is carried out in the separation and collection step includes solid-liquid separation and washing of the bacterial cell. The production method can include, in addition to the above-described steps, other steps. Examples of the other steps include a resting bacterial cell reaction step (which can be carried out after the culturing step, preferably after the separation and collection step), a drying step (which can be carried out after the separation and collection step and the resting bacterial cell reaction step), and a bacterial cell crushing step (which can be performed after the separation and collection step, the resting bacterial cell reaction step, or the drying step).

Examples of the medium that can be used in the culturing step include those used for expansion culture (preculture medium) and those used for production culture (main culture medium). The main culture medium can be prepared by additionally incorporating additives into basically a medium used as the preculture medium. With regard to the form of the medium, the medium is preferably a liquid medium, but may be an agar medium. The medium typically contains a nitrogen source, minerals, and the like in addition to a carbon source.

Examples of the carbon source include carbohydrates and carbohydrate materials. Examples of the carbohydrate include saccharides (monosaccharides, disaccharides, oligosaccharides), polysaccharides, and sugar alcohols. Examples of the carbohydrate include lactose, sucrose, glucose, starch, xylitol, and dextrose. The carbohydrate material may be any organic composition containing a carbohydrate, and examples thereof include foods such as milk and processed products thereof (nonfat dry milk, whey, milk powder, condensed milk and the like), soy milk and processed products thereof (soy milk hydrolysate and the like), cereals, fruit, and vegetables. Examples of the milk include those derived from any mammal such as a cow, a goat, sheep, a buffalo, a camel, a llama, a donkey, a yak, a horse or a reindeer. The carbohydrate may be isolated, or contained in a carbohydrate material. For example, the fructose (carbohydrate) may be used as it is contained in fruit (carbohydrate material). These carbon sources may be used alone, or may be used in combination of two or more thereof. Among these carbon sources, glucose is preferable.

The concentration of the carbon source in the medium is not limited, and may be appropriately set according to the type of medium, the culture method and the like, and is, for example, 0.5 to 4 w/w%, preferably 1 to 3 w/w%, and more preferably 1.5 to 2.5 w/w%.

As the nitrogen source, any inorganic nitrogen source or organic nitrogen source can be used. Examples thereof include: proteins such as yeast extract (beer yeast and the like), meat extract, and casein; protein hydrolysates such as peptone (protease peptone and the like), peptides; ammonium salts (ammonium citrate and the like), and nitrogen-containing salts such as nitric acid salts. These nitrogen sources may be used alone, or may be used in combination of two or more thereof.

The concentration of the nitrogen source in the medium is not limited, and may be appropriately set according to the type of medium, the culture method and the like, and is, for example, 0.3 to 4 w/w%, preferably 0.5 to 3 w/w%, and more preferably 1 to 2 w/w% in the case of the protein; for example, 0.1 to 2 w/w%, preferably 0.3 to 1.8 w/w%, and more preferably 0.5 to 1.5 w/w% in the case of the peptide; and for example, 0.03 to 1.5 w/w%, preferably 0.05 to 1 w/w%, and more preferably 0.1 to 0.5 w/w% in the case of the nitrogen-containing salt.

Examples of the mineral include manganese (for example, manganese salts such as manganese sulfate), zinc, iron, sodium (for example, sodium salts such as sodium acetate), potassium (for example, potassium salts such as dipotassium hydrogen sulfate and dipotassium hydrogen phosphate), magnesium (for example, magnesium salts such as magnesium sulfate), calcium, phosphorus (for example, a phosphoric acid salts such dipotassium hydrogen phosphate), sulfur (for example, sulfuric acid salts such as manganese sulfate, potassium hydrogen sulfate, or magnesium sulfate), trace elements, and the like. These minerals may be used alone, or may be used in combination of two or more thereof. Among these minerals, manganese, sodium, magnesium and potassium are preferable.

The concentration of the mineral in the medium is not limited, and may be appropriately set according to the type of medium, the culture method and the like, and is, for example, 0.001 to 0.01 w/w%, and preferably 0.003 to 0.008 w/w% in the case of the manganese salt; for example, 0.05 to 1.5 w/w%, and preferably 0.1 to 1 w/w% in the case of the sodium salt; for example, 0.001 to 0.02 w/w%, and preferably 0.005 to 0.015 w/w% in the case of the magnesium salt; for example, 0.05 to 1 w/w%, and preferably 0.1 to 0.5 w/w% in the case of the potassium salt; for example, 0.05 to 1 w/w%, and preferably 0.1 to 0.5 w/w% in the case of the phosphoric acid salt; and for example, 0.001 to 0.04 w/w%, and preferably 0.005 to 0.02 w/w% in the case of the sulfuric acid salt.

The medium may contain, in addition to the above-described components, other components such as vitamins (for example, vitamin B family), surfactants (nonionic surfactants (Tween and the like), anionic surfactants (SDS and the like), and the like), antibacterial agents (triclosan and the like), and antibiotics (monensin and the like). These other components may be used alone, or may be used in combination of two or more thereof. Among these other components, surfactants are preferable, and nonionic surfactants are more preferable.

The concentration of the other component in the medium is not limited, and may be appropriately set according to the type of the other component, the type of medium, the culture method and the like, and when a surfactant is contained, the concentration of the surfactant is, for example, 0.01 to 0.5 w/w%, and preferably 0.05 to 0.3 w/w%.

The culture conditions are not limited as long as they are conditions under which the genus Fructobacillus lactic acid bacterium can grow.

The culture temperature may be any optimal temperature for a genus Fructobacillus lactic acid bacterium to be cultured, and is, for example, 26 to 40°C, preferably 27 to 38°C, more preferably 28 to 36°C, and still more preferably 29 to 34°C. The culture time may be appropriately set according to the type of genus Fructobacillus lactic acid bacterium to be actually cultured, and is, for example, 4 to 48 hours, preferably 8 to 36 hours, and more preferably 12 to 24 hours.

For the operation during culture, it does not matter whether or not it is necessary to stir the culture solution during culture. As a specific example of a culture procedure, the above-described culture can be performed as production culture (main culture) for a certain period of time, and preceded by expansion culture (preculture) performed in a small amount of a medium (1/6 to 1/4 times the amount of the main culture medium on a volume basis). The culture conditions for the preculture may be appropriately set according to the type of genus Fructobacillus lactic acid bacterium or the like, and the above-described conditions can be adopted. In the main culture, a culture obtained from the preculture can be inoculated into the main culture medium to an OD 660 of, for example, 0.01 to 0.04, preferably 0.01 to 0.03.

In the separation and collection step, solid-liquid separation is performed on the culture solution by filtration using filter paper, centrifugation, decantation, screw pressing, roller pressing, rotary drum screening, belt screening, vibration screening, multi-plate vibration filtration, vacuum dewatering, pressure dewatering, belt pressing, dewatering by centrifugal concentration, multi-disc dewatering, or the like, and the resulting bacterial cells can be washed.

In the resting bacterial cell step, the content ratio of nicotinamide mononucleotide in the lactic acid bacterium can be increased.

The liquid that can be used in the resting bacterial cell reaction step is not limited as long as it allows a lactic acid bacterium belonging to the genus Fructobacillus to undergo a resting bacterial cell reaction. For example, water, a buffer solution, an organic solvent, or the like can be used.

The pH of the liquid used for the resting bacterial cell reaction is, for example, 4.0 to 10.0, preferably pH 5.0 to 9.0, and more preferably 5.5 to 7.5.

When the liquid used for the resting bacterial cell reaction is a buffer solution, examples of the buffer solution include acetate buffer solutions, phosphate buffer solutions, borate buffer solutions, carbonate buffer solutions, citrate buffer solutions, Tris buffer solutions, and HEPES buffer solutions.

More specific examples of the buffer solution include KHC₈H₄O₄-NaOH (pH4.0), CH₃COOH-CH₃COONa (pH4.0), MES-NaOH (pH5.0), CH₃COOH-CH₃COONa (pH5.0), KH₂PO₄-K₂HPO4 (pH6.0), MES-NaOH (pH6.0), KH₂PO₄-K₂HPO₄ (pH7.0), PIPES-NaOH (pH7.0), HEPES-NaOH (pH8.0), H₃BO₄-NaOH (pH8.0), CHES-NaOH (pH9.0), H₃BO₄-NaOH (pH9.0), H₂CO₃-NaHCO₃ (pH10.0), and CHES-NaOH (pH10.0). CH₃COOH-CH₃COONa, KH₂PO₄-K₂HPO4 and H₃BO₄-NaOH are preferable, and KH₂PO₄-K₂HPO4 is more preferable.

When the liquid used for the resting bacterial cell reaction is an organic solvent, examples of the organic solvent include aromatic compounds such as benzene and benzonitrile, ketones such as acetone, acetylacetone and methyl ethyl ketone, fatty acid esters such as ethyl acetate, butyl acetate, ethyl butyrate and ethyl formate, ethers such as diethyl ether, dipropyl ether, dibutyl ether, tetrahydrofuran and 1,4-dioxane, halogenated hydrocarbons such as dichloromethane, chloroform and dichloroethane, 1,2-propanediol, 1,2-butanediol, 1,3-butanediol, 1,4-butanediol, 2,3-butanediol, 1,2-hexanediol, 1,6-hexanediol, 1,2-pentanediol, 1,5-pentanediol, 2-methyl -2,4-pentanediol and 3-methyl-1,5-pentanediol, and alcohols such as alcohols having a linear or branched alkyl having 1 to 7 carbon atoms, cyclohexanol, 3-methoxy-3-methyl-1-butanol and 3-methoxy-1-butanol.

The liquid used for the resting bacterial cell reaction is preferably water or a buffer solution among the above-described liquids.

The reaction temperature in the resting bacterial cell reaction is, for example, 21 to 37°C, and preferably 24 to 28°C. The reaction time is, for example, 0.1 to 24 hours, and preferably 6 to 12 hours.

The resting bacterial cell reaction can be carried out by suspending a lactic acid bacterium belonging to the genus Fructobacillus in the above-described liquid, followed by standing, stirring or shaking.

For carrying out the resting bacterial cell reaction, the pH of a culture solution containing a lactic acid bacterium belonging to the genus Fructobacillus may be adjusted to be in the range of 4.0 to 10.0, preferably pH 5.0 to 9.0, and more 5.5 to 7.5, instead of using the above-described liquid.

In the drying step, the bacterial cells can be dried using a drying method in a step of freeze drying, shelf drying, spray drying or the like.

In the bacterial cell crushing step, dried bacterial cells can be crushed by any method. In the bacterial cell crushing step, a part of bacterial cell components may be removed from the crushed bacterial cells by a method other than extraction.

### [1-2-2. Culture of lactic acid bacterium]

When the active ingredient is in the form of a culture of a lactic acid bacterium, the active ingredient includes, together with the lactic acid bacterium, a medium component containing a product from the lactic acid bacterium. The culture of the lactic acid bacterium may be a culture produced using one of the predetermined lactic acid bacteria alone, a culture produced using two or more of the lactic acid bacteria in combination plurality of lactic acid bacteria, or a mixture of a culture produced using one lactic acid bacterium and a culture produced using another lactic acid bacterium.

In the medium components contained in the culture, a part of the medium components used for culturing the lactic acid bacterium may be removed. The lactic acid bacterium contained in the culture may be either a living bacterium or a dead bacterium. In the case of a dead bacterium, bacterial cells may be crushed. In the case where bacterial cells are crushed, a part of the bacterial cell components may be removed.

The culture of the lactic acid bacterium can be obtained by a production method including the step of culturing one or more of the predetermined lactic acid bacteria. The production method can include, in addition to the above-described steps, other steps. Other steps may include a step of removing a part of the medium components, a step of subjecting lactic acid bacteria to a resting bacterial cell reaction, and/or a drying step. Details of the steps are as described in "1-2-1. Lactic acid bacteria" above.

### [1-2-3. Culture supernatant of lactic acid bacterium]

When the active ingredient is in the form of a culture supernatant of a lactic acid bacterium, the active ingredient includes a medium component containing a product from the lactic acid bacterium. The culture supernatant of the lactic acid bacterium may be a culture supernatant produced using one of the predetermined lactic acid bacteria alone, a culture supernatant produced using two or more of the lactic acid bacteria in combination plurality of lactic acid bacteria, or a mixture of a culture supernatant produced using one lactic acid bacterium and a culture supernatant produced using another lactic acid bacterium.

In the medium components contained in the culture supernatant, a part of the medium components used for culturing the lactic acid bacterium may be removed.

The culture supernatant of the lactic acid bacterium can be obtained by a production method including the steps of: culturing one or more of the predetermined lactic acid bacteria, and separating and collecting the culture supernatant. The production method can include, in addition to the above-described steps, other steps. Other steps may include a step of removing a part of the medium components, a step of subjecting lactic acid bacteria to a resting bacterial cell reaction, and/or a drying step. Details of the steps are as described in "1-2-1. Lactic acid bacteria" above.

### [1-2-4. Extract of lactic acid bacterium]

When the active ingredient is in the form of an extract of a lactic acid bacterium, the active ingredient is a multi-component composition obtained by subjecting the lactic acid bacterium or the culture of the lactic acid bacterium to extraction treatment. Examples of the extraction solvent used in preparation of the extract include water; monohydric lower alcohols having 1 to 4 carbon atoms, such as ethanol and isopropanol; polyhydric alcohols such as 1,3-butylene glycol, propylene glycol and glycerin; polar solvents such as mixed liquids thereof, and water is preferable from the viewpoint of further effectively improving the collagen production promoting effect, the hyaluronic acid production promoting effect and/or the epidermal keratinocyte activating effect. That is, in a preferred form, the extract of a lactic acid bacterium is a water extract of a lactic acid bacterium. The temperature condition during extraction is preferably room temperature (for example, 5 to 35°C, preferably 20 to 30°C). Examples of the specific method for preparing an extract include a method in which a suspension containing the lactic acid bacterium or a culture of the lactic acid bacterium or a pulverized product thereof in an extraction solvent, and performing solid-liquid separation on the suspension to obtain an extraction liquid (extract liquid); and a method in which the extraction liquid is dried as necessary to obtain powder (extract powder).

### [1-3. Components contained in active ingredient]

A lactic acid bacterium belonging to the genus Fructobacillus, a culture of the lactic acid bacterium, a culture supernatant of the lactic acid bacterium and an extract of the lactic acid bacterium, each of which is an active ingredient for use in the present invention, all contain not only NMN and/or NAD) produced by the lactic acid bacterium but also other metabolites (other than NMN and NAD) and/or bacterial cell components. The excellent collagen production promoting effect, hyaluronic acid production promoting effect and epidermal keratinocyte activating effect according to the present invention are exhibited by the above-mentioned components in combination.

When the active ingredient for use in the present invention contains at least NAD and may contain NMN, the ratio to NMN is not limited, and the ratio of NMN is, for example, 5 parts by weight or less per 1 part by weight of NAD. From the viewpoint of further effectively improving the collagen production promoting effect, the hyaluronic acid production promoting effect, and/or the epidermal keratinocyte activating effect, the ratio of NMN is preferably 3 parts by weight or less, more preferably 2.3 parts by weight or less, still more preferably 1.7 parts by weight or less, even more preferably 1 part by weight or less, further more preferably 0.5 parts by weight or less, particularly preferably 0.1 parts by weight or less, and most preferably 0.07 parts by weight or less per 1 part by weight of NAD. In a more preferred form, the ratio of NMN is preferably 0.06 parts by weight or less per 1 part by weight of NAD when the lactic acid bacterium is Fructobacillus fructosus, the ratio of NMN is preferably 0.06 parts by weight or less, and more preferably 0.05 parts by weight or less per 1 part by weight of NAD when the lactic acid bacterium is Fructobacillus durionis, and the ratio of NMN is preferably 0.06 parts by weight or less, more preferably 0.05 parts by weight or less, still more preferably 0.04 parts by weight or less, and even more preferably 0.03 parts by weight or less per 1 part by weight of NAD when the lactic acid bacterium is Fructobacillus tropaeoil.

The ratio of NMN is, for example, 10 mol or less per 1 mol of NAD. From the viewpoint of further effectively improving the collagen production promoting effect, the hyaluronic acid production promoting effect, and/or the epidermal keratinocyte activating effect, the ratio of NMN is preferably 6 mol or less, more preferably 4.6 mol or less, still more preferably 3.4 mol or less, even more preferably 2 mol or less, further more preferably 1 mol or less, particularly preferably 0.2 mol or less, and most preferably 0.14 mol or less per 1 mol of NAD. In a more preferred form, the ratio of NMN is preferably 0.12 mol or less per 1 part by weight of NAD when the lactic acid bacterium is Fructobacillus fructosus, the ratio of NMN is preferably 0.12 mol or less, and more preferably 0.1 mol or less per 1 part by weight of NAD when the lactic acid bacterium is Fructobacillus durionis, and the ratio of NMN is preferably 0.12 mol or less, more preferably 0.1 mol or less, still more preferably 0.08 mol or less, and even more preferably 0.06 mol or less per 1 mol of NAD when the lactic acid bacterium is Fructobacillus tropaeoil.

When the active ingredient for use in the present invention contains NMN and NAD, the ratio to NMN is not limited, and the ratio of NMN is, for example, 0.005 parts by weight or more, and more preferably 0.01 parts by weight per 1 part by weight of NAD. In a more preferred form, the ratio of NMN is preferably 0.02 parts by weight or more, more preferably 0.03 parts by weight or more, still more preferably 0.04 parts by weight or more, even more preferably 0.045 parts by weight or more, and further more preferably 0.05 parts by weight or more per 1 part by weight of NAD when the lactic acid bacterium is Fructobacillus fructosus, the ratio of NMN is preferably 0.02 parts by weight or more, more preferably 0.03 parts by weight or more, and still more preferably 0.04 parts by weight or more per 1 part by weight of NAD when the lactic acid bacterium is Fructobacillus durionis, and the ratio of NMN is preferably 0.015 parts by weight or more, more preferably 0.018 parts by weight or more, still more preferably 0.02 parts by weight or more, and even more preferably 0.023 parts by weight or more per 1 part by weight of NAD when the lactic acid bacterium is Fructobacillus tropaeoil.

The ratio of NMN is, for example, 0.01 mol or more, and preferably 0.02 mol or more per 1 mol of NAD. In a more preferred form, the ratio of NMN is preferably 0.04 mol or more, more preferably 0.06 mol or more, still more preferably 0.08 mol or more, even more preferably 0.09 mol or more, and further more preferably 0.1 mol or more per 1 mol of NAD when the lactic acid bacterium is Fructobacillus fructosus, the ratio of NMN is preferably 0.04 mol or more, more preferably 0.06 mol or more, and still more preferably 0.08 mol or more per 1 part by weight of NAD when the lactic acid bacterium is Fructobacillus durionis, and the ratio of NMN is preferably 0.03 mol or more, more preferably 0.035 mol or more, still more preferably 0.04 mol or more, and even more preferably 0.046 mol or more per 1 part by weight of NAD when the lactic acid bacterium is Fructobacillus tropaeoil.

### [1-4. Content of active ingredient]

The amount of the active ingredient contained in each of the collagen production promoter, the hyaluronic acid production promoter and the epidermal keratinocyte activator of the present invention is not limited, and can be appropriately determined according to a collagen production promoting effect, a hyaluronic acid production promoting effect or an epidermal keratinocyte activating effect to be imparted.

A specific example of the content of the active ingredient contained in each of the collagen production promoter, the hyaluronic acid production promoter and the epidermal keratinocyte activator of the present invention is, for example, 0.00001 to 10 wt%, and preferably 0.00001 to 1 wt% on a dry weight basis in terms of a Fructobacillus lactic acid bacterium (that is, this value refers, in the case where the active ingredient is a lactic acid bacterium or a culture of the lactic acid bacterium, to the dry weight of the active ingredient, refers, in the case where the active ingredient is a culture supernatant of a lactic acid bacterium, to the dry weight of lactic acid bacterium used for acquisition of the culture supernatant, and refers, in the case where the active ingredient is an extract, to the dry weight of a lactic acid bacterium used for acquisition of the extract).

As another specific example of the content of the active ingredient contained in each of the collagen production promoter, the hyaluronic acid production promoter and the epidermal keratinocyte activator of the present invention is, for example, 0.05 µM or more or 0.1 µM or more, preferably 0.15 µM or more, more preferably 0.4 µM or more, still more preferably 0.75 µM or more or 0.9 µM or more, even more preferably 1.5 µM or more or 1.7 µM or more, and further more preferably 3 µM or more or 3.5 µM or more in terms of a total amount of NMN and NAD. The upper limit of the total amount of NMN and NAD contained in each of the collagen production promoter, the hyaluronic acid production promoter and the epidermal keratinocyte activator of the present invention is not limited, and is, for example, 10 µM or less, 8 µM or less, 6 µM or less, 4 µM or less, or 2 µM or less.

### [2. Other components]

The collagen production promoter, the hyaluronic acid production promoter and the epidermal keratinocyte activator of the present invention may, but are not required to, contain, in addition to the above-described active ingredient, other pharmacological components as necessary. Examples of the pharmacological components include anti-inflammatory agents, antioxidants, bactericides, refreshing agents, vitamins, and mucopolysaccharides.

The collagen production promoter, the hyaluronic acid production promoter and the epidermal keratinocyte activator of the present invention may, but are not required to, contain a base and/or additives as necessary for obtaining a desired preparation form. The base and/or additives are not limited as long as they are pharmaceutically acceptable, and examples thereof include aqueous bases such as water and a monovalent lower alcohols having 1 to 4 carbon atoms (ethanol, isopropanol and the like); oily bases such as naturally occurring oil (vegetable oil, animal oil, and processed oil thereof), mineral oil, ester oil, fatty acid alkyl esters, fatty acid, fatty acid ester and higher alcohols; surfactants; polyhydric alcohols (glycerin, propylene glycol, dipropylene glycol, 1,3-butylene glycol and the like); and additives such as refreshing agents, preservatives, flavoring agents, coloring agents, thickening agents, pH adjusters, wetting agents, stabilizers, antioxidants, ultraviolet absorbers, chelating agents, pressure sensitive adhesives, buffering agents, solubilizing agents, solubilizers and preservatives.

### [3. Preparation form and product classification]

The preparation form of each of the collagen production promoter, the hyaluronic acid production promoter and the epidermal keratinocyte activator of the present invention is not limited, and may be any of a liquid form, a semi-solid form (cream, gel, ointment or paste), a solid form (granule, fine granule, powder, tablet or capsule) and the like. Each of the collagen production promoter, the hyaluronic acid production promoter and the epidermal keratinocyte activator of the present invention may be a non-emulsified preparation such as an aqueous preparation or an oily preparation, or an emulsified preparation such as an oil-in-water emulsified preparation or a water-in-oil emulsified preparation.

Examples of the product classification of the collagen production promoter, the hyaluronic acid production promoter and the epidermal keratinocyte activator of the present invention include food or beverage products, cosmetic products, and pharmaceutical products.

The food or beverage product is not limited, and examples thereof include beverages such as fermented milk (drink yogurt and the like), lactic acid bacterium beverages, milk beverages (milky coffee, fruit milk and the like), tea beverages (green tea, black tea, oolong tea and the like), fruit and vegetable beverages (beverages containing fruit juice from orange, apple, grape or the like, or vegetable juice from tomato, carrot or the like), alcoholic beverages (beer, low-malt beer, wine and the like), carbonated beverages, soft beverages, and water-based beverages; and processed food products such as fermented milk (set type yogurt, soft yogurt and the like), confectionery, ready-to-eat food products and seasoning.

Examples of the food or drink product include functional food products. The functional food product means a food product having a certain functionality for living organisms, and examples thereof include foods with health claims such as special health foods (including conditional special health foods) and foods with nutrient function claims, foods with functional claims, foods for special dietary uses, nutritional supplementary foods, supplements (those in various dosage forms, for example, tablets, coated tablets, sugar-coated tablets, capsules and solutions), and beauty diets (for example, diet foods). Further, the functional food product may be food for special dietary uses, such as patient food, powdered milk for pregnant women and nursing women, formula milk for infants, food product for the aged, and nursing food.

Examples of the cosmetic product include external preparations including external preparations for skin and external preparations for mucous membrane, and more specific examples include basic cosmetics such as lotion, emulsion, cream, essence, gel, packs, sheet masks and lip cream; skin cleansers such as facial wash, makeup removers (including cleansing agents), exfoliating cleansers and body shampoos; body care cosmetics such as sunscreen agents, body gel, body rubbing agents, antiperspirants, deodorants, depilatories and bath additives; makeup cosmetics such as foundation, face powder, lipstick, blush, eye shadow, eyeliner, mascara and eyebrow powder; nail cosmetics such as manicure and nail removers; hair cosmetics such as hair styling agents, shampoos, conditioners, rinse and hair tonics; cosmetics for oral use such as liquid dentifrice, toothpaste, mouth wash solutions and mouth sprays.

Examples of the pharmaceutical product include oral preparations, and external preparations including external preparation for skin and an external preparation for mucous membrane (including quasi-drugs). Examples of the dosage form of the oral preparation include tablets, coated tablets, sugar-coated tablets, capsules and solutions, and examples of the dosage form of the external preparation include solutions (including lotions, sprays, aerosols and emulsions), foams, ointments, creams, gels and patches.

Among these product classifications, external preparations are preferable, and external preparations for skin are more preferable from the viewpoint of further enhancing the collagen production promoting effect, the hyaluronic acid production promoting effect and the epidermal keratinocyte activating effect.

### [4. Applications]

The collagen production promoter, the hyaluronic acid production promoter and the epidermal keratinocyte activator of the present invention are used in applications for promotion of the production of collagen, promotion of the production of hyaluronic acid and the effect of activating epidermal keratinocytes, respectively.

Examples of the application for promotion of the production of collagen include applications in which an action of connecting cells, an action of maintaining the strength of blood vessels and/or muscles, and/or an action of forming skin, bone and/or mucous membrane are utilized. More specific examples include prevention or remediation of sagging and/or wrinkles of the skin; treatment of roughness and/or wounds of the skin; prevention or treatment of osteoporosis, arthritis and/or tenosynovitis; and the like, which are applications in which conditions or symptoms in tissues that require improvement of the above-mentioned actions (skin, bone, mucous membrane and the like). Therefore, a genus Fructobacillus lactic acid bacterium, a culture of the lactic acid bacterium, a culture supernatant of the lactic acid bacterium and/or an extract of the lactic acid bacterium can also be used as an active ingredient of an agent for preventing or treating sagging and/or wrinkles of the skin; an agent for treating roughness and/or wounds of the skin; and an agent for preventing or treating osteoporosis, arthritis and/or tenosynovitis, etc.

Examples of the application for promotion of the production of hyaluronic acid include applications in which an action of retaining moisture between cells, and an action of lubrication and buffering in a living organism are utilized. More specific examples include moisturization of the skin; prevention or remediation of sagging of the skin; prevention or treatment of arthritis; treatment of keratoconjunctive epithelium disorder; retention of the anterior chamber during ophthalmic surgery (for example, cataract and corneal transplant surgery); and the like, which are prevention, remediation or treatment of conditions or symptoms in tissues that require improvement of the above-mentioned actions (skin, bone, mucous membrane and the like). Therefore, a genus Fructobacillus lactic acid bacterium, a culture of the lactic acid bacterium, a culture supernatant of the lactic acid bacterium and/or an extract of the lactic acid bacterium can also be used as an active ingredient of a skin moisturizing agent; an agent for preventing or remedying sagging of the skin; an agent for preventing or treating arthritis; an agent for treating keratoconjunctive epithelium disorder; and an agent for retaining the anterior chamber during ophthalmic surgery (for example, cataract and corneal transplant surgery).

Examples of the application for the effect of activating epidermal keratinocytes include applications in which an action of proliferating epidermal keratinocytes is, that is, applications in which an action of promoting a turnover of the skin is utilized. More specific examples include suppression of keratin thickening; remediation of skin dullness caused by a decrease in activity of keratinocytes; promotion of regeneration of the skin; treatment of wounds; treatment of roughness of the skin; moisturization of the skin; skin lightening; improvement of the skin texture; treatment of acne and/or pimples; and the like, which are prevention, remediation or treatment of skin symptoms caused by stagnation of a turnover of the epidermis. Therefore, a genus Fructobacillus lactic acid bacterium, a culture of the lactic acid bacterium, a culture supernatant of the lactic acid bacterium and/or an extract of the lactic acid bacterium can also be used as an active ingredient of a keratin thickening suppressing agent; an agent for remedying skin dullness; a skin regeneration promoting agent; an agent for treating wounds; an agent for treating roughness of the skin; a skin moisturizing agent; a skin-lightening agent; a skin texture improving agent; and an agent for treating acne and/or pimples.

### [5. Dosage]

When the collagen production promoter, the hyaluronic acid production promoter and the epidermal keratinocyte activator of the present invention are internally administered, they can be internally administered one to five times a day in an amount of, for example, 0.01 to 200 mg/kg/day, preferably 0.1 to 20 mg/kg/day in terms of an active ingredient.

When the collagen production promoter, the hyaluronic acid production promoter and the epidermal keratinocyte activator of the present invention are externally administered, they can be externally administered one to five times a day in an application amount of, for example, 0.0001 to 0.2 mg/cm², preferably 0.001 to 0.02 mg/cm², per administration in terms of an active ingredient.

### EXAMPLES

Hereinafter, the present invention will be described in more detail by showing examples, which should not be construed as limiting the present invention.

### [Preparation of sample]

### (1) Culture of genus Fructobacillus lactic acid bacterium (preparation of sample A)

A Fructobacillus fructosus NBRC 3516 strain was inoculated into 30 ml of MRS medium (preculture medium) manufactured by Difco Laboratories, and was subjected to expansion culture in a static state at 30°C for 24 hours. The obtained culture solution was inoculated into 1 L of MRS medium (main culture medium) to an OD 660 of 0.02, and cultured with stirring at a pH of 6.0 to 7.0 at 30°C for 12 hours.

The obtained culture solution was centrifuged to collect bacterial cells. The collected bacterial cells were washed with 1 L of a 0.85 w/w% KCl aqueous solution. The washed bacterial cells were centrifuged again to collect bacterial cells. The collected bacterial cells were killed and then freeze-dried to obtain lactic acid bacterium powder (sample A).

The amount of production of each of nicotinamide mononucleotide (NMN) and nicotinamide adenine dinucleotide (NAD) in the collected bacterial cells was measured under HPLC analysis conditions described later. The results of the measurement showed that the weight ratio of NMN was 0.055 parts by weight per 1 part by weight of NAD.

### (2) Preparation of lactic acid bacterium extraction liquid A1 (preparation of extraction liquid of sample A)

20 g of the lactic acid bacteria powder (sample A) was finely ground in a mortar and suspended in 1 L of sterile water to prepare a suspension. The suspension was stirred at room temperature (about 25°C) for 1 hour, and centrifuged (12,000 rpm × 10 min), and the supernatant was then filtered through a 0.2 µm filter to obtain a lactic acid bacterium extraction liquid A1.

The amount of production of each of nicotinamide mononucleotide (NMN) and nicotinamide adenine dinucleotide (NAD) in the obtained lactic acid bacterium extraction liquid A1 was measured under HPLC analysis conditions described later. The results of the measurement showed that the weight ratio of NMN was 0.054 parts by weight per 1 part by weight of NAD.

### (3) Culture of genus Fructobacillus lactic acid bacterium and resting bacterial cell reaction (preparation of sample B)

A Fructobacillus fructosus NBRC 3516 strain was inoculated into 30 ml of MRS medium (preculture medium) manufactured by Difco Laboratories, and was subjected to expansion culture in a static state at 30°C for 24 hours. The obtained culture solution was inoculated into 1 L of MRS medium (main culture medium) to an OD 660 of 0.02, and cultured with stirring at a pH of 6.0 to 7.0 at 30°C for 12 hours.

The obtained culture solution was centrifuged to collect bacterial cells. The collected bacterial cells were washed with 1 L of a 0.85 w/w% KCl aqueous solution. The washed bacterial cells were centrifuged again to collect bacterial cells. The collected bacterial cells were suspended in 200 ml of ion-exchanged water and stirred at a pH of 6.0 to 7.0 at 25°C for 8 hours to carry out a resting bacterial cell reaction. After completion of the resting bacterial cell reaction, the reaction mixture was centrifuged to collect bacterial cells. The collected bacterial cells were washed with 1 L of a 0.85 w/w% KCl aqueous solution. The washed bacterial cells were centrifuged again to collect bacterial cells. The collected bacterial cells were killed and then freeze-dried to obtain lactic acid bacterium powder after resting bacterial cell reaction (sample B).

The amount of production of each of nicotinamide mononucleotide (NMN) and nicotinamide adenine dinucleotide (NAD) in the collected bacterial cells was measured under HPLC analysis conditions described later. The results of the measurement showed that the weight ratio of NMN was 1.55 parts by weight per 1 part by weight of NAD.

### (4) Preparation of lactic acid bacterium extraction liquid B1 (preparation of extraction liquid of sample B)

20 g of the lactic acid bacteria powder (sample B) was suspended in 1 L of sterile water to prepare a suspension. The suspension was stirred at room temperature (about 25°C) for 1 hour, and centrifuged (12,000 rpm × 10 min), and the supernatant was filtered through a 0.2 µm filter to obtain a lactic acid bacterium extraction liquid B1.

The amount of production of each of nicotinamide mononucleotide (NMN) and nicotinamide adenine dinucleotide (NAD) in the obtained lactic acid bacterium extraction liquid B1 was measured under HPLC analysis conditions described later. The results of the measurement showed that the weight ratio of NMN was 0.86 parts by weight per 1 part by weight of NAD.

### (5) Preparation of lactic acid bacterium extraction liquids of other Fructobacillus lactic acid bacteria (lactic acid bacterium extraction liquid C1, lactic acid bacterium extraction liquid D1 and lactic acid bacterium extraction liquid E1)

Powder of each lactic acid bacterium was prepared in the same manner as in "(1) Preparation of powder of genus Fructobacillus lactic acid bacterium (sample A)" above except that a Fructobacillus durionis RD 011727 strain, a Fructobacillus tropaeoil RD 012353 strain, or a Fructobacillus tropaeoil RD 012354 strain was used as the lactic acid bacterium. Using the obtained powder of each lactic acid bacterium, an extraction liquid was prepared in the same manner as in "(2) Preparation of lactic acid bacterium extraction liquid A1 (preparation of extraction liquid of sample A)" above.

Specifically, an extraction liquid of the Fructobacillus durionis RD 011727 strain (lactic acid bacterium extraction liquid C1) was prepared from powder of the strain, an extraction liquid of the Fructobacillus tropaeoil RD 012353 strain (lactic acid bacterium extraction liquid D1) was prepared from powder of the strain, and an extraction liquid of the Fructobacillus tropaeoil RD 012354 strain (lactic acid bacterium extraction liquid E1) was prepared from powder of the strain.

The amount of production of each of nicotinamide mononucleotide (NMN) and nicotinamide adenine dinucleotide (NAD) contained in each of the lactic acid bacterium extraction liquid C1, the lactic acid bacterium extraction liquid D1 and the lactic acid bacterium extraction liquid E1 was measured under HPLC analysis conditions described later. The results of the measurement showed that the weight ratio of NMN was 0.0421 parts by weight per 1 part by weight of NAD in the lactic acid bacterium extraction liquid C1, the weight ratio of NMN was 0.0202 parts by weight per 1 part by weight of NAD in the lactic acid bacterium extraction liquid D1, and the weight ratio of NMN was 0.0250 parts by weight per 1 part by weight of NAD in the lactic acid bacterium extraction liquid E1.

### (MRS medium composition)

| | |
|---|---|
| 2 w/w% | Glucose |
| 1 w/w% | Protease peptone |
| 1 w/w% | Beef extract |
| 0.5 w/w% | Yeast extract |
| 0.2 w/w% | Ammonium citrate |
| 0.1 w/w% | Tween 80 |
| 0.5 w/w% | Sodium acetate |
| 0.01 w/w% | Magnesium sulfate |
| 0.005 w/w% | Manganese sulfate |
| 0.2 w/w% | Dipotassium hydrogen phosphate |

### (HPLC analysis conditions) NMN measurement method

Column: DaisoPak SP-100-5-ODS-P (4.6 × 150 mm) × 2
Column temperature: 25°C
Eluent: 75 mM ammonium phosphate aqueous solution (pH 6.0)
Flow rate: 0.6 ml/min
Detector: UV detector (260 nm)
Detection time: 14.5 min

### (HPLC analysis conditions) NAD measurement method

Column: DAISOPAK SP-100-5-ODS-P (4.6 × 250 mm) × 2
Column temperature: 25°C
Eluent: 75 mM ammonium phosphate aqueous solution (pH 6.0) : methanol = 95 : 5 (w : w)
Flow rate: 0.7 mL/min
Detector: UV detector (260 nm)
Detection time: 33 min

### [Test Example 1: Promoting effect of lactic acid bacterium powder (sample A) on production of collagen and hyaluronic acid]

### [1 Overview]

In the present test example, the addition of lactic acid bacterium powder (sample A) to human dermal fibroblast cells significantly increased the collagen production ratio per number of cells in comparison with the control, and significantly increased the hyaluronic acid production ratio per number of cells in comparison with the control. Therefore, it was suggested that the lactic acid bacterium powder (sample A) enhanced the production of collagen and the production of hyaluronic acid in the fibroblast cells.

### [2 Test purpose]

Collagen has an action of connecting cells, an action of maintaining the strength of blood vessels and muscles, and an action of forming skin, bone and mucous membrane. Hyaluronic acid has an action of retaining moisture between cells, and an action of lubrication and buffering in a living organism. In the present test example, dermal fibroblast cells were used to verify the effect of lactic acid bacterium powder on the production of the above-mentioned components.

### [3 Outline of test]

Lactic acid bacterium powder was used as a test product. Dermal fibroblast cells were cultured in a medium containing the lactic acid bacterium powder, and the amount of collagen and hyaluronic acid in the culture supernatant were measured by an enzyme-linked immuno sorbent assay (ELISA) method. On the basis of the measured values, the action of promoting the production of collagen and hyaluronic acid by the lactic acid bacterium powder was evaluated.

### [4 Materials and test methods]

### [4-1 Cells]

Human neonatal-derived dermal fibroblast cell line NB1 RGB cells (Riken BRC, Japan) were cultured using a CO₂ incubator (CO₂ concentration: 5%, 37°C), and the obtained culture was used in the present test example.

### [4-2 Medium]

Eagle's minimal essential medium (EMEM, Cat No. 051-07615, Wako, Japan) containing 10.0% (v/v) fetal bovine serum FBS, Cat No. SH 30071.03, Hyclone, UK) and 1.0% (v/v) antifungal agent (Antibiotic-Antimycotic 100 X, Cat No. 15240 - 062, Invitrogen, USA) was used.

### [4-3 Test product]

The sample A was diluted using EMEM containing 0.5% FBS, thereby preparing three types of test products having different concentrations (10 µg/mL, 25 µg/mL, 500 µg/mL). The specific composition of the test product is as in Table 1. In Table 1, the composition of the test product is shown by the NAD amount and the NMN amount as a final concentration in the culture solution of fibroblast cells. EMEM containing 0.5% FBS was used as a control.

**[Table 1]**

| | Fructobacillus fructosus NBRC 3516 strain lactic acid bacterium powder (sample A) | | |
|---|---|---|---|
| Concentration of test product | 10 (µg/ml) | 25 (µg/ml) | 50 (µg/ml) |
| Amount of bacterial cell-derived NAD in culture solution (µM) | 0.164 | 0.41 | 0.82 |
| Amount of bacterial cell-derived NMN in culture solution (µM) | 0.018 | 0.045 | 0.09 |

| | | | |
|---|---|---|---|
| NMN/NAD weight ratio = 0.055 | | | |

### [4-4 Test setup]

In calculation of the number of living cells, the amount of collagen production and the amount of hyaluronic acid production, three wells of a 96-well plate were used for each treatment group. In calculation of the number of living cells, a 96-well plate Cat No. 3595 (Corning, USA) was used, and in calculation of the amount of collagen production and the amount of hyaluronic acid production, a 96-well plate Cat No. 3855 (Thermo scientific, USA) was used. One test product group and one control group were provided in each plate. Test related operations, including preparation of a test product, were carried out at room temperature unless otherwise stated.

### [4-5 Test operations]

### [4-5-1 Cell culture]

1) NB1RGB cells were seeded at a density of 1.0 × 10⁴ cells/well in a 96-well plate, and cultured in a CO₂ incubator for 24 hours. For preventing drying during culture, 200 µL of phosphate buffer saline (PBS (-), Cat No. 198601, Nissui, Japan) was added to wells that were not used in the test.
2) After 24 hours, the medium was removed, and 100 µL of each of the test product and the control was added to a 96-well plate and cultured in a CO₂ incubator for 48 hours.
3) After 48 hours, the culture supernatant was dispensed to a new 96-well plate, and the amount of collagen and the amount of hyaluronic acid in the culture supernatant were measured by the ELISA method shown in 4-5-3 and 4-5-4 described later, thereby evaluating the action of the test product on the production of collagen and hyaluronic acid by the test product. The number of living cells in the 96-well plate, from which the culture supernatant had been removed, was measured by the method shown in 4-5-2 described later.

### [4-5-2 Measurement of number of living cells]

1) Each well of the 96-well plate, from which the medium had been removed, was gently washed once with 200 µL of PBS (-) heated to 37°C.
2) A solution of 3-(4,5-dimethylthiazol-2-yl)-2,5-diphenyltetrazolium bromide (MTT, CAS No. 298-93-1, Sigma-Aldrich, USA) at 0.5 mg/mL was added in an amount of 200 µL per well, and the cells were cultured in a CO₂ incubator for 2 hours.
3) After completion of the culture, the MTT solution was removed, and washing was performed with 200 µL of PBS (-). For solubilizing the insoluble formazan formed, 200 µL of 2-propanol (CAS No. 67-63-0, Kanto Chemical, Japan) containing 0.04 N hydrochloric acid (CAS No. 7647 01-0, Kanto Chemical, Japan) was added, followed by standing in a light-shielding environment at room temperature for 1 hour.
4) The 96-well plate was shaken at 270 rpm for 10 seconds to uniformly disperse the dye in the well, and the absorbance at 570 nm (OD 570) was then measured using a microplate reader (SPARKR 10 M, TECAN, Switzerland).

### [4-5-3 Evaluation of collagen production promoting action]

The effect of the test product on the amount of production of type 1 collagen, which is said to impart tension and elasticity to the skin, was evaluated by a direct ELISA method.
1) To a high adsorption-type 96-well plate, a collagen standard solution and a culture supernatant dispensed in "4-5-1 cell culture" were added in an amount of 100 µL per well, followed by standing overnight at 4°C.
2) The microplate was washed twice with 200 µL of 0.05% Tween 20 in PBS (-) (Wash buffer, Tween 20: Cas No. 9005-64-5, Sigma-Aldrich, USA), 200 µL of 1% bovine serum albumin (BSA, Cat No. PRL 68700-50 G, Proliant, USA) in PBS was added, followed by standing at room temperature for 1 hour.
3) After 1 hour, washing was performed twice with 200 µL of Wash buffer, and 100 µL of a biotin-bound anti-collagen type I antibody (Cat No. 600-406-103, ROCKLAND, USA) solution was added, followed by standing at room temperature for 1 hour.
4) After 1 hour, washing was performed four times with 200 µL of Wash buffer, and 100 µL of a Streptavidin-HRP (Cat No. CJ30H-1, Agilent Technologies, USA) solution was added, followed by standing at room temperature for 30 minutes.
5) After 30 minutes, washing was performed four times with 200 µL of Wash buffer and once with 200 µL of ultrapure water (CAS No. 7732-18-5, Wako, Japan).
6) 2,2'-Azinobis(3-ethylbenzothiazoline-6-sulfonic acid)diammonium salt (ABTS, Cat No. 5110-0010, KPL, USA) was added in an amount of 100 µL per 1 well, followed by standing at room temperature for 5 minutes.
7) The 96-well plate was shaken at 270 rpm for 30 seconds to uniform the dye in the well, and the absorbance at 405 nm (OD 405) was then measured using a microplate reader.
8) The OD 405 of each of the control and the test product was divided by the OD 570 measured in "4-5-2 Measurement of number of living cells", and the thus-obtained value was determined as a collagen production ratio per cell. The collagen production ratio per cell (%) for the test product when the collagen production ratio per cell for the control group was defined as 100% was calculated.

### [4-5-4 Evaluation of hyaluronic acid production promoting action]

The effect of the test product on the amount of production of hyaluronic acid, which is said to hold moisture between collagens or elastins, was evaluated by a sandwich ELISA method.
1) A solution of hyaluronan binding protein (HABP, Cat No. BC 40, Hokuto, Japan) at 5 µg/mL was added in an amount of 100 µL to a high adsorption-type 96-well plate, followed by standing at 4 °C for 24 hours.
2) The HABP solution was removed, and washing was performed three times with 200 µL of a solution of 0.05% Tween 20 in 1.5 M NaCl solution (Wash buffer, USA NaCl: Cas No. 7647-14-5, Wako, Japan).
3) 100 µL of a 5% BSA solution was added thereto, followed by standing overnight at 4°C.
4) The BSA solution was removed, washing was performed twice with 200 µL of Wash buffer, and the plate was then dried.
5) To a 96-well plate, a culture supernatant diluted 100-fold with PBS (-) containing 0.5 M NaCl, 0.02% Tween 20 and 1% BSA was added in an amount of 100 µL per well, followed by standing at room temperature for 2 hours.
6) After 2 hours, washing was performed four times with 200 µL of Wash buffer, 100 µL of a biotin-labeled HABP (Cat No. BC41, Hokudo, Japan) solution was added, followed by standing at room temperature for 30 minutes.
7) After 30 minutes, washing was performed four times with 200 µL of Wash buffer and once with 200 µL of ultrapure water, and 100 µL of a Streptavidin-HRP solution was added, followed by standing at room temperature for 30 minutes.
8) After 30 minutes, washing was performed four times with 200 µL of Wash buffer and once with 200 µL of ultrapure water, and ABTS was added in an amount of 100 µL per well, followed by standing at room temperature for 10 minutes.
9) The 96-well plate was shaken at 270 rpm for 30 seconds to uniform the dye in the well, and the absorbance at 405 nm (OD 405) was then measured using a microplate reader.
10) The OD 405 of each of the control and the test product was divided by the OD 570 measured in "4-5-2 Measurement of number of living cells", and the thus-obtained value was determined as a hyaluronic acid production ratio per cell. The hyaluronic acid production ratio per cell (%) for the test product when the hyaluronic acid production ratio per cell for the control group was defined as 100% was calculated.

### [6 Test results]

### [6-1-1 Evaluation of type 1 collagen production promoting action]

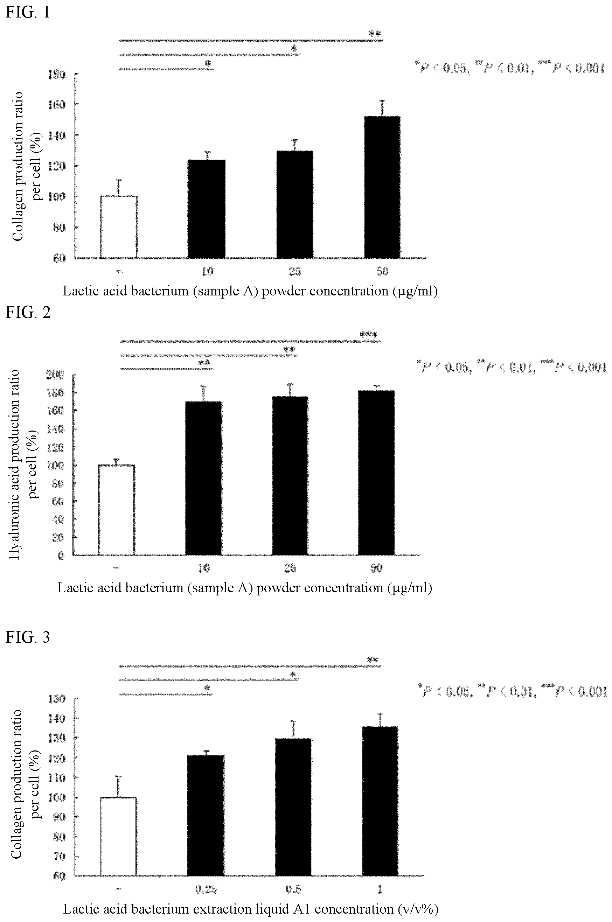

The average value and the standard deviation of the collagen production ratio per cell (%) for the test product are shown in Fig. 1.

### [6-2 Evaluation of hyaluronic acid production promoting action]

The average value and the standard deviation of the hyaluronic acid production ratio per cell (%) for the test product are shown in Fig. 2.

### [Test Example 2: Promoting effect of lactic acid bacterium extraction liquid A1 on production of collagen and hyaluronic acid]

In the present test example, the addition of the lactic acid bacterium extraction liquid A1 to human dermal fibroblast cells significantly increased the collagen production ratio per cell in comparison with the control, and significantly increased the hyaluronic acid production ratio per cell in comparison with the control. Therefore, it was suggested that the lactic acid bacterium extraction liquid A1 enhanced the production of collagen and the production of hyaluronic acid in the fibroblast cells.

In the present test example, the same test as in Test Example 1 was conducted except that the test product was changed to the following test products from those described in "4-3 Test product" in Test Example 1.

The lactic acid bacterium extraction liquid A1 was diluted using EMEM containing 0.5% FBS, thereby preparing three types of test products having different concentrations (0.25% (v/v), 0.5% (v/v), 1% (v/v)). The specific composition of the test product is as in Table 2. In Table 2, the composition of the test product is shown by the NAD amount and the NMN amount as a final concentration in the culture solution of fibroblast cells. EMEM containing 0.5% FBS was used as a control.

**[Table 2]**

| | Fructobacillus fructosus NBRC 3516 strain lactic acid bacterium extraction liquid A1 | | |
|---|---|---|---|
| Concentration of test product | 0.25 (v/v%) | 0.5 (v/v%) | 1 (v/v%) |
| Amount of bacterial cell-derived NAD in culture solution (µM) | 0.84 | 1.68 | 3.36 |
| Amount of bacterial cell-derived NMN in culture solution (µM) | 0.09 | 0.18 | 0.36 |

| | | | |
|---|---|---|---|
| NMN/NAD weight ratio = 0.054 | | | |

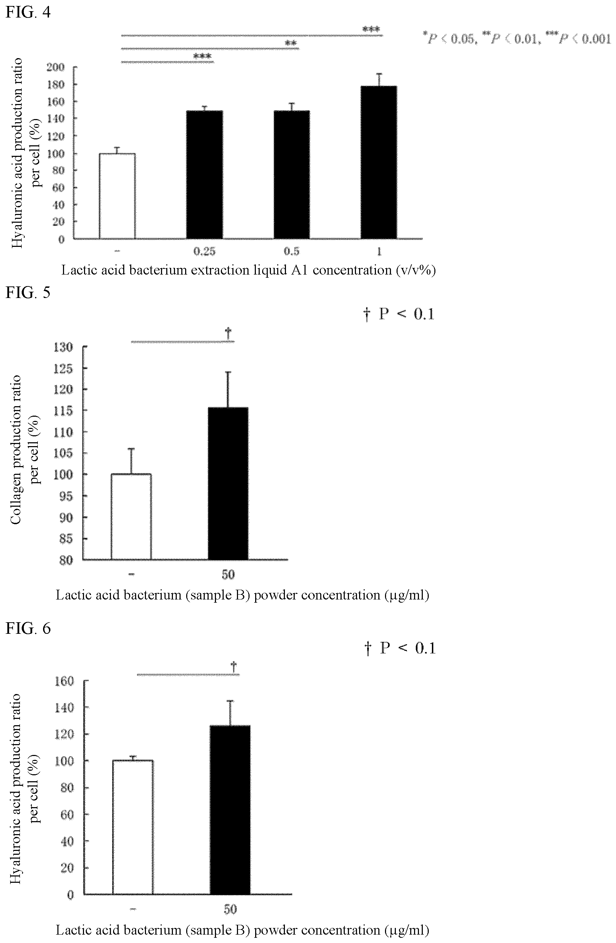

The average value and the standard deviation of the collagen production ratio per cell (%) for the test product when the collagen production ratio per cell for the control is defined as 100% are shown in Fig. 3. The average value and the standard deviation of the hyaluronic acid production ratio per cell (%) for the test product when the hyaluronic acid production ratio per cell for the control is defined as 100% are shown in Fig. 4.

### [Test Example 3: Promoting effect of lactic acid bacterium powder (sample B) on production of collagen and hyaluronic acid]

In the present test example, the addition of lactic acid bacterium powder (sample B) to human dermal fibroblast cells significantly increased the collagen production ratio per number of cells in comparison with the control, and significantly increased the hyaluronic acid production ratio per number of cells in comparison with the control. Therefore, it was suggested that the lactic acid bacterium powder (sample B) enhanced the production of collagen and the production of hyaluronic acid in the fibroblast cells.

In the present test example, the same test as in Test Example 1 was conducted except that the test product was changed to the following test products from those described in "4-3 Test product" in Test Example 1.

The sample B was diluted to 50 µg/mL with EMEM containing 0.5% FBS, thereby preparing a test product. The specific composition of the test product is as in Table 3. In Table 3, the composition of the test product is shown by the NAD amount and the NMN amount as a final concentration in the culture solution of fibroblast cells. EMEM containing 0.5% FBS was used as a control.

**[Table 3]**

| | Fructobacillus fructosus NBRC 3516 strain lactic acid bacterium powder (sample B) |
|---|---|
| Concentration of test product | 50 (µg/ml) |
| Amount of bacterial cell-derived NAD in culture solution (µM) | 0.15 |
| Amount of bacterial cell-derived NMN in culture solution (µM) | 0.46 |

| | |
|---|---|
| NMN/NAD weight ratio = 1.55 | |

The average value and the standard deviation of the collagen production ratio per cell (%) for the test product when the collagen production ratio per cell for the control group is defined as 100% are shown in Fig. 5. The average value and the standard deviation of the hyaluronic acid production ratio per cell (%) for the test product when the hyaluronic acid production ratio per cell for the control is defined as 100% are shown in Fig. 6.

### [Test Example 4: Promoting effect of lactic acid bacterium extraction liquid B1 on production of collagen and hyaluronic acid]

In the present test example, the addition of the lactic acid bacterium extraction liquid B1 to human dermal fibroblast cells significantly increased the collagen production ratio per cell in comparison with the control, significantly increased the hyaluronic acid production ratio in comparison with the control, and significantly increased the hyaluronic acid production ratio per cell in comparison with the control. Therefore, it was suggested that the lactic acid bacterium extraction liquid B1 enhanced the production of collagen and the production of hyaluronic acid in the fibroblast cells.

In the present test example, the same test as in Test Example 1 was conducted except that the test product was changed to the following test products from those described in "4-3 Test product" in Test Example 1.

The lactic acid bacterium extraction liquid B 1 was diluted using a medium, thereby preparing two types of test products having different concentrations (0.5% (v/v), 1% (v/v)). The specific composition of the test product is as in Table 4. In Table 4, the composition of the test product is shown by the NAD amount and the NMN amount as a final concentration in the culture solution of fibroblast cells. A medium was used as a control.

**[Table 4]**

| | Fructobacillus fructosus NBRC 3516 strain lactic acid bacterium extraction liquid B1 | |
|---|---|---|
| Concentration of test product | 0.5 (v/v%) | 1 (v/v%) |
| Amount of bacterial cell-derived NAD in culture solution (µM) | 0.44 | 0.88 |
| Amount of bacterial cell-derived NMN in culture solution (µM) | 0.75 | 1.5 |

| | | |
|---|---|---|
| NMN/NAD weight ratio = 0.86 | | |

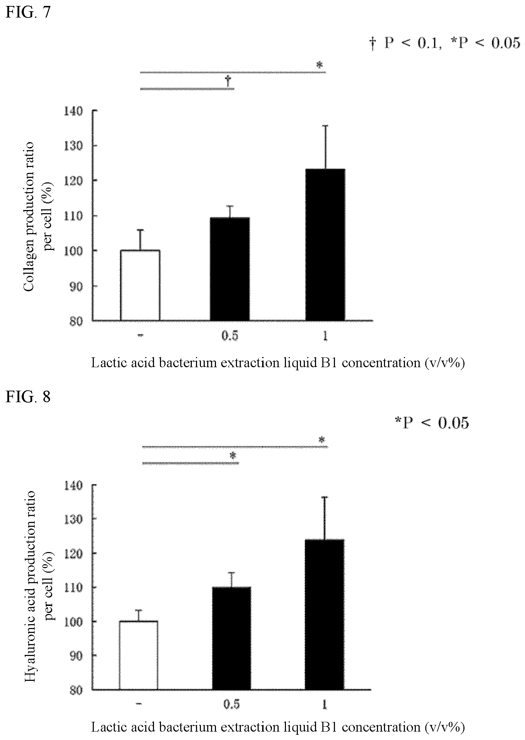

The average value and the standard deviation of the collagen production ratio per cell (%) for the test product when the collagen production ratio per cell for the control is defined as 100% are shown in Fig. 7. The average value and the standard deviation of the hyaluronic acid production ratio per cell (%) for the test product when the hyaluronic acid production ratio per cell for the control is defined as 100% are shown in Fig. 8.

### [Test Example 5: Human epidermal keratinocyte activating effect of lactic acid bacterium extraction liquid]

### [Test Example 5-1: Human epidermal keratinocyte activating effect of lactic acid bacterium extraction liquid A1]

### [1 Overview]

In the present test example, it was suggested that the addition of the lactic acid bacterium extraction liquid A1 to human epidermal keratinocytes increased the cell proliferation ratio in comparison with the control.

### [2 Test purpose]

The turnover of the epidermis is involved in the skin barrier function and maintenance of the moisture content. The turnover cycle of the skin is a rate-limiting step for the proliferation (that is, activation) of epidermal keratinocytes. In the present test example, the effect of the lactic acid bacterium extraction liquid on cell activation was verified using human epidermal keratinocytes.

### [3 Outline of test]

A lactic acid bacterium extraction liquid was used as a test product. Human epidermal keratinocytes were cultured in a medium containing lactic acid bacterium extraction liquid, and a degree of cell proliferation which indicates cell activation was measured by the absorbance at 450 nm (OD 450). On the basis of the obtained OD 450, the action of cell activation by the lactic acid bacterium extraction liquid was evaluated.

### [4 Materials and test methods]

### [4-1 Cells]

Human epidermal keratinocytes PSVK1 (JCRB Cell Bank. JCRB 1093, Cell Originator: Yamamoto, S) were cultured using a CO₂ incubator (CO₂ concentration: 5%, 37 °C), and used in the present test example.

### [4-2 Medium]

An additive (KGM-Gold Single Quots (Lonza)) was added to KMG-Gold base medium (Lonza) to prepare a medium for use in the present test example.

### [4-3 Test product]

The lactic acid bacterium extraction liquid A1 was diluted with the medium to prepare a test product at 1.0% (v/v). A medium was used as a control.

### [4-4 Test setup]

In calculation of the cell activation, three wells of a 96-well plate were used for each treatment group. One test product group and one control group were provided in each plate, and the test was conducted. Test related operations, including preparation of a test product, were carried out at room temperature unless otherwise stated.

### [4-5 Test operations]

### [4-5-1 Cell culture]

1) PSVK1 cells were seeded at a density of 5.0 × 10³ cells/well in a 96-well plate, and cultured in a CO₂ incubator for 24 hours. For preventing drying during culture, 200 µL of PBS (-) was added to wells that were not used in the test.
2) After 24 hours, the medium was removed, and 100 µL of each of the test product and the control was added to a 96-well plate and cultured in a CO₂ incubator for 48 hours.
3) After 48 hours, the culture supernatant was removed, the number of cells in the 96-well plate was evaluated by a method shown in 4-5-2 described later, and the human epidermal keratinocyte activating action of the test product was evaluated.

### [4-5-2 Evaluation of human epidermal keratinocyte activating action]

1) Each well of the 96-well plate, from which the medium had been removed, was gently washed once with 200 µL of PBS (-) heated to 37°C.
2) To the 96-well plate, 100 µL of a medium was added. A CCK-8 solution (Cell Counting Kit-8, Dojindo, Japan) was added in an amount of 10 µL per well, and a color reaction was carried out in a CO₂ incubator for 3 hours.
3) After completion of the reaction, the absorbance at 450 nm (OD 450) was measured using a microplate reader (Infinite^{(R)} 200 PRO, TECAN, Switzerland). The higher the measured value of OD 450, the larger the number of cells.
4) The relative value of OD 450 in the test product addition group when OD 450 in the control group was defined as 100% was calculated as a cell proliferation ratio of human epidermal keratinocytes for the test article, that is, a human epidermal keratinocyte activation ratio (%). The results are shown in Table 5.

### [Test Example 5-2: Human epidermal keratinocyte activating effect of lactic acid bacterium extraction liquid C1]

In the present test example, it was suggested that the addition of the lactic acid bacterium extraction liquid C1 to human epidermal keratinocytes increased the cell proliferation ratio in comparison with the control.

Specifically, except that the test product was changed to the lactic acid bacterium extraction liquid C1, the same procedure as in Test Example 5-1 was carried out to calculate a human epidermal keratinocyte activation ratio (%). The results are shown in Table 5.

### [Test Example 5-3: Human epidermal keratinocyte activating effect of lactic acid bacterium extraction liquid D1]

In the present test example, it was suggested that the addition of the lactic acid bacterium extraction liquid D1 to human epidermal keratinocytes increased the cell proliferation ratio in comparison with the control.

Specifically, except that the test product was changed to the lactic acid bacterium extraction liquid D1, the same procedure as in Test Example 5-1 was carried out to calculate a human epidermal keratinocyte activation ratio (%). The results are shown in Table 5.

### [Test Example 5-4: Human epidermal keratinocyte activating effect of lactic acid bacterium extraction liquid E1]

In the present test example, it was suggested that the addition of the lactic acid bacterium extraction liquid E1 to human epidermal keratinocytes increased the cell proliferation ratio in comparison with the control.

Specifically, except that the test product was changed to the lactic acid bacterium extraction liquid E1, the same procedure as in Test Example 5-1 was carried out to calculate a human epidermal keratinocyte activation ratio (%). The results are shown in Table 5.

**[Table 5]**

| | Lactic acid bacterium extraction liquid A1 | Lactic acid bacterium extraction liquid C1 | Lactic acid bacterium extraction liquid D1 | Lactic acid bacterium extraction liquid E1 |
|---|---|---|---|---|
| Strain of origin | Fructobacillus fructosus NBRC 3516 strain | Fructobacillus durionis RD011727 strain | Fructobacillus tropaeoil RD012353 strain | Fructobacillus tropaeoil RD012354 strain |
| Concentration of test product | 1.0% (v/v) | | | |
| Amount of bacterial cell-derived NAD in culture solution (µM) | 3.36 | 1.72 | 1.79 | 1.560 |
| Amount of bacterial cell-derived NMN in culture solution (µM) | 0.36 | 0.14 | 0.07 | 0.080 |
| NMN/NAD weight ratio | 0.054 | 0.0421 | 0.0202 | 0.025 |
| Human epidermal keratinocyte activation ratio | 135% | 109% | 126% | 116% |

## Claims

1. A collagen production promoter comprising a lactic acid bacterium belonging to the genus Fructobacillus, a culture of the lactic acid bacterium, a culture supernatant of the lactic acid bacterium, and/or an extract of the lactic acid bacterium.

2. The collagen production promoter according to claim 1, wherein the lactic acid bacterium is Fructobacillus fructosus.

3. The collagen production promoter according to claim 1, wherein the lactic acid bacterium is a Fructobacillus fructosus NBRC 3516 strain.

4. The collagen production promoter according to claim 1, comprising nicotinamide adenine dinucleotide and optionally including a nicotinamide mononucleotide, in which a content of the nicotinamide mononucleotide is 5 parts by weight or less per 1 part by weight of the nicotinamide adenine dinucleotide.

5. A hyaluronic acid production promoter comprising a lactic acid bacterium belonging to the genus Fructobacillus, a culture of the lactic acid bacterium, a culture supernatant of the lactic acid bacterium, and/or an extract of the lactic acid bacterium.

6. The hyaluronic acid production promoter according to claim 5, wherein the lactic acid bacterium is Fructobacillus fructosus.

7. The hyaluronic acid production promoter according to claim 5, wherein the lactic acid bacterium is a Fructobacillus fructosus NBRC 3516 strain.

8. The hyaluronic acid production promoter according to claim 5, comprising nicotinamide adenine dinucleotide and optionally including a nicotinamide mononucleotide, in which a content of the nicotinamide mononucleotide is 5 parts by weight or less per 1 part by weight of the nicotinamide adenine dinucleotide.

9. An epidermal keratinocyte activator comprising a lactic acid bacterium belonging to the genus Fructobacillus, a culture of the lactic acid bacterium, a culture supernatant of the lactic acid bacterium, and/or an extract of the lactic acid bacterium.

10. The epidermal keratinocyte activator according to claim 9, wherein the lactic acid bacterium is selected from the group consisting of Fructobacillus fructosus, Fructobacillus durionis, and Fructobacillus tropaeoil.

11. The epidermal keratinocyte activator according to claim 9, wherein the lactic acid bacterium is selected from the group consisting of a Fructobacillus fructosus NBRC 3516 strain, a Fructobacillus durionis RD 011727 strain, a Fructobacillus tropaeoil RD 012353 strain, and a Fructobacillus tropaeoil RD 012354 strain.

12. The epidermal keratinocyte activator according to claim 9, comprising nicotinamide adenine dinucleotide and optionally including a nicotinamide mononucleotide,
wherein a content of the nicotinamide mononucleotide is 5 parts by weight or less per 1 part by weight of the nicotinamide adenine dinucleotide.

13. A food or beverage product, a cosmetic product or a pharmaceutical product comprising the collagen production promoter according to claim 1.

14. A food or beverage product, a cosmetic product or a pharmaceutical product comprising the hyaluronic acid production promoter according to claim 5.

15. A food or beverage product, a cosmetic product or a pharmaceutical product comprising the epidermal keratinocyte activator according to claim 9.

16. An agent for preventing or remedying sagging and/or wrinkles of skin,
an agent for treating roughness and/or wounds of the skin,
an agent for preventing or treating osteoporosis, arthritis and/or tenosynovitis,
a skin moisturizing agent,
an agent for treating keratoconjunctive epithelium disorder,
an agent for retaining the anterior chamber during ophthalmic surgery,
a keratin thickening suppressing agent,
an agent for remedying skin dullness,
a skin regeneration promoting agent,
a skin-lightening agent,
a skin texture improving agent, or
an agent for remedying acne and/or pimples,
the agent comprising a lactic acid bacterium belonging to the genus Fructobacillus, a culture of the lactic acid bacterium, a culture supernatant of the lactic acid bacterium, and/or an extract of the lactic acid bacterium.
